# EUROPÄISCHE PATENTANMELDUNG

(11) **EP 0 543 288 A1**
(43) Veröffentlichungstag der Anmeldung: **26.05.1993**
(21) Anmeldenummer: 92119445.2
(22) Anmeldetag: 13.11.1992
(51) Int. Cl.: C07C 43/12, C07C 41/01, C08G 65/32

(54) **Verfahren zur Herstellung von perfluorierten Ethern**

(30) Priorität: 16.11.1991 DE 4137783
(71) Anmelder: HOECHST AKTIENGESELLSCHAFT, D-65926 Frankfurt am Main (DE)
(72) Erfinder: Meyer, Matthias, Dr., W-2000 Hamburg 65 (DE)

(57) **Zusammenfassung**

Die Erfindung betrifft ein Verfahren zur Herstellung von perfluorierten Ethern der Formel (I)

R-CF₂-CF₃ (I),

worin R den Rest CF₃(CF₂)₂-O-[CF(CF₃)CF₂-O]ₙ- bedeutet, in dem n eine ganze Zahl von 0-60 ist, aus Verbindungen der Formel (II)

R-[CF(CF₃)CF₂-O-]CHF-CF₃ (II),

in dem man diese in Gegenwart von mindestens einer der Verbindungen AlCl₃, AlBr₃, AlF₃ auf 150-350°C erhitzt.

## Beschreibung

Die Erfindung betrifft ein Verfahren zur Herstellung von perfluorierten Ethern der Formel (I)

R-CF₂-CF₃ (I)

worin R den Rest CF₃(CF₂)₂-O-[CF(CF₃)CF₂O]ₙ- bedeutet, in dem n eine ganze Zahl von 0-60 ist, aus Verbindungen der Formel (II)

R-[CF(CF₃)CF₂-O-]CHF-CF₃ (II).

Perfluorether der Formel (I) zeichnen sich durch eine hohe thermische Beständigkeit aus, sie sind unbrennbar und chemisch stabil, auch gegenüber Angriffen starker Oxidationsmittel. Die in Formel (II) verwendeten eckigen Klammern dienen nur zum besseren Verständnis und können im Prinzip auch weggelassen werden, so daß sich die gleichwertige Formel R-CF(CF₃)CF₂-O-CHF-CF₃ ergibt.

Aufgrund ihrer besonderen physikalischen Eigenschaften, nämlich günstigem Viskositätsverhalten in weiten Temperaturbereichen, hohen Siedepunkten bei gleichzeitig niedrigen Stockpunkten, geringer Oberflächenspannung und extremen dielektrischen Eigenschaften, finden perfluorierte Polyether als Inertflüssigkeiten, Dielektrika, Wärmetauschmittel, Hydrauliköle und Schmiermittel in Gegenwart von stark aggressiv wirkenden Medien vielseitige Anwendungen.

In EP-A-0 154 297 ist die Oligomerisation von Hexafluorpropenoxid (HFPO) in Gegenwart von CsF und Polyethylenglykoldimethylethern, insbesondere Tetraethylenglykoldimethylether, beschrieben. Das Roholigomer fällt dabei im allgemeinen als Gemisch mit einer Molmassenverteilung von ca. 300-20000 g/mol an. Es besteht aus Perfluoretheracylfluoriden der Formel R-[CF(CF₃)CF₂-O-]CF(CF₃)-COF, wobei R die oben angegebene Bedeutung hat.

Die genannten Acylfluoride sind hydrolyseempfindlich, spalten bei hoher Temperatur aggressive und toxische fluorierte Bruchstücke ab und sind daher als Inertflüssigkeiten nicht einsetzbar.

In der deutschen Patentanmeldung P 4120508.1 wird die Umsetzung dieser Perfluoretheracylfluoride mit AlCl₃ und/oder AlBr₃ zu perfluorierten Ethern der Formel (I) beschrieben.

Gemäß EP-A-0 154 297 werden dagegen die Acylfluoride zu Carbonsäuren hydrolysiert, in die Salze überführt und diese anschließend in Gegenwart von alkalischen Medien decarboxyliert. Auf diesem Wege erhält man jedoch nur unvollständig fluorierte Ether der Formel R-[CF(CF₃)CF₂-O-]CHF-CF₃, die zwar nicht hydrolyseempfindlich sind, jedoch bei höheren Temperaturen in Gegenwart von Luftsauerstoff instabil sind. In US-PS 3 595 925 wird die Umsetzung dieser Verbindungen unter Verwendung äquimolarer Mengen von SbF₅ zu den perfluorierten Verbindungen der Formel (I) gemäß der Gleichung

R-[CF(CF₃)CF₂-O-]CHF-CF₃ + SbF₅ ---> R-[CF(CF₃)CF₂-O-]CF₂-CF₃ + HF + SbF₃

beschrieben. Nachteil dieses Verfahrens ist der Einsatz des kostspieligen Antimonpentafluorids.

Überraschenderweise wurde gefunden, daß sich die genannten unvollständig fluorierten Ether in Gegenwart von Aluminiumhalogeniden in perfluorierte Ether mit einer um 3 C-Atome verkürzten Kette umwandeln lassen.

Gegenstand der Erfindung ist ein Verfahren zur Herstellung von perfluorierten Ethern der Formel (I)

R-CF₂-CF₃ (I),

worin R den Rest CF₃(CF₂)₂-O-[CF-(CF₃)CF₂-O-]ₙ- bedeutet, in dem n eine ganze Zahl von 0-60 ist, aus Verbindungen der Formel (II)

R-[CF(CF₃)CF₂-O-]CHF-CF₃

dadurch gekennzeichnet, daß man die Verbindungen der Formel (II) in Gegenwart von mindestens einer der Verbindungen AlCl₃, AlBr₃, AlF₃ auf 150-350°C erhitzt.

Die Ausgangsverbindungen der Formel (II) werden beispielsweise in einem Rührkolben unter Inertgasatmosphäre mit mindestens einer der Verbindungen AlCl₃ AlF₃, AlBr₃ auf Reaktionstemperatur erhitzt. Vorzugsweise legt man jedoch die Verbindungen (II) vor, erhitzt diese und fügt dann das Aluminiumhalogenid hinzu. Ein Lösungsmittel ist nicht erforderlich.

Die zugesetzten Mengen an Aluminiumhalogenid betragen im allgemeinen 1 bis 100 Mol.-%, bezogen auf die eingesetzte Menge der Verbindung (II), vorzugsweise 5-10 Mol.%.

Die Reaktionstemperatur beträgt 150-300°C, vorzugsweise 200-280°C, insbesondere 240-260°C.

Es werden die fluorierten Verbindungen der allgemeinen Formel (II)

R-[CF(CF₃)CF₂-O-]CHF-CF₃

eingesetzt, worin R den Rest CF₃(CF₂)₂-O-[CF(CF₃)CF₂-O-]ₙ- bedeutet und n eine ganze Zahl von 0-60 ist. Der Wert von n kann durch die Temperatur bei der Oligomerisation von HFPO gemäß EP-A-0 154 297 gesteuert werden. Je tiefer die Temperatur gewählt wird, umso höher ist der Wert von n. Besonders interessant sind Werte von n = 10 bis n = 60. Bei der Oligomerisation von HFPO entsteht ein Gemisch von Acylfluoriden der Formel R-[CF(CF₃)CF₂-O-]CF(CF₃)-COF. Die Verbindungen der Formel (II) erhält man gemäß EP-A-0 154 297 durch Hydrolyse und Decarboxylierung dieser Acylfluoride mit wäßriger Kaliumhydroxidlösung in Ethylenglykol bei 175°C. Die Verbindungen der Formel (II) werden durch Extraktion vom Ethylenglykol, Filtration vom Kaliumfluorid und anschließende Trocknung gereinigt, bevor sie in das erfindungsgemäße Verfahren eingesetzt werden.

Die Reaktion der Verbindungen (II) kann IR- und ¹⁹F-NMR-spektroskopisch verfolgt werden und ist beendet, wenn im IR-Spektrum die CH-Bande bei etwa 3000 cm⁻¹ verschwunden ist und im ¹⁹F-NMR-Spektrum die Signale der -CHFCF₃-Endgruppe nicht mehr sichtbar sind.

Nach Reaktionsende wird durch Filtration vom Aluminiumhalogenid-Feststoff abgetrennt.

Das farblose Filtrat kann ohne weitere Reinigungsschritte der Fraktionierung in Siedebereiche durch Kurzwegdestillation zugeführt werden.

### Beispiele

### Versuchsbericht Darstellung des Ausgangsmaterials (II) gemäß EP-A-0 154 297.

Eine Lösung von 20 g CsF in 50 ml Tetraethylenglykoldimethylether (Tetraglyme) und 56 g Hexafluorpropylenoxid wurden unter Stickstoffatmosphäre in einem 4 l V-4A Autoklaven vorgelegt. Zusätzlich wurden 250 ml ^{R}Frigen F113 zur Verdünnung hinzugefügt. Bei einer Temperatur von - 5°C wurden dann 4000 g Hexafluorpropylenoxid über einen Zeitraum von 8 Stunden unter guter Durchmischung eingeleitet. Nach Reaktionsende wurde auf Raumtemperatur erwärmt und das entstandene Acylfluorid-Roholigomer unter N₂-Atmosphäre abgelassen.

3000 g dieses Acylfluorid-Oligomers R-[CF(CF₃)CF₂-O-]CF(CF₃)-COF mit einem mittleren Molekulargewicht von 3000 g mol⁻¹ wurden in einem 4 l-Dreihalskolben vorgelegt. In einer Lösung aus 500 ml Diethylenglykol und 200 ml Wasser wurden 60 g KOH gelöst und langsam zum Acylfluorid-Oligomer hinzugegeben. Die Reaktionslösung wurde zunächst 3 h bei einer Temperatur von 90°C gerührt und anschließend wurde auf 175°C für etwa 6 h erhitzt. Nach Reaktionsende wurde die untere, fluororganische Phase, die die Verbindung R-[CF(CF₃)CF₂-O-]CHF-CF₃ enthielt, von der Glykol-Wasser-Phase getrennt. Die fluororganische Phase wurde mit 1000 ml Frigen F113 verdünnt und anschließend mit Wasser, dann mit verdünnter HCl und nochmals mit Wasser gewaschen. Nach Abtrennung des Restgehaltes an Wasser, Frigen F113 und Diethylenglykol wurden bei 200°C unter vermindertem Druck die Verbindungen (II) R-[CF(CF₃)CF₂-O-]CHF-CF₃ erhalten.

### Beispiel 1

300 g (0.23 Mol) eines Gemisches von Verbindungen der Formel (II) mit einem mittleren Molekulargewicht von 1300 g mol⁻¹ (erhalten durch Kurzwegdestillation des gemäß dem Versuchsbericht hergestellten Ausgangsmaterials) wurden mit 3 g AlCl₃ (= 22 mMol) versetzt und unter N₂-Atmosphäre auf 250°C erhitzt. Die Reaktion wurde ¹⁹F-NMR-spektroskopisch verfolgt und war nach 7 h beendet. Das Produkt wurde durch Filtration vom AlCl₃-Feststoff abgetrennt. Es wurden 245 g eines wasserklaren Filtrats erhalten (Ausbeute: 92%).

Die Charakterisierung des Produkts erfolgte mit Hilfe der IR- und ¹⁹F-NMR-Spektroskopie. Die Elementaranalyse ergab für die Elemente C,F,O die nach Formel (I) zu erwartenden Werte.

### Beispiel 2

300 g eines wie in Beispiel 1 erhaltenen Gemischs von Verbindungen der Formel (II) wurden unter Inertgasatmosphäre auf 225°C erhitzt und 2 g AlCl₃ portionsweise hinzugegeben. Die Reaktion war nach 4 h beendet. Nach Aufarbeitung wie im Beispiel 1 wurden 255 g eines Oligomers der Formel (I) erhalten (Ausbeute: 96%). Die Charakterisierung des Produktes erfolgte wie im Beispiel 1 beschrieben.

### Beispiel 3

300 g (0.1 Mol) eines Gemisches von Verbindungen der Formel (II) mit einem mittleren Molekulargewicht von 3000 gmol⁻¹ wurden mit 1.3 g AlCl₃ versetzt und auf 270°C erhitzt. Die Reaktion war nach 2 h beendet. Die Aufarbeitung des Produkts erfolgte wie in den Beispielen 1 und 2. Es wurden 268 g an perfluorierten Verbindungen (I) erhalten (Ausbeute: 94%). Die Charakterisierung des Produkts erfolgte wie im Beispiel 1.

### Beispiel 4

Es wurde analog zu Beispiel 3 gearbeitet. Anstelle von AlCl₃ wurden jedoch 0.9 g AlF₃ hinzugefügt. Die Reaktion war nach 3 h beendet.

Nach Aufarbeitung, wie im Beispiel 3 beschrieben, wurden 265 g perfluoriertes Produkt (I) erhalten (Ausbeute: 90%). Die Charakterisierung des Produkts erfolgte wie im Beispiel 1.

### Beispiel 5

Es wurde analog zu Beispiel 3 gearbeitet, jedoch wurden anstelle von AlCl₃ 2.7 g AlBr₃ eingesetzt. Es wurden 236 g an perfluorierten Verbindungen (I) erhalten (Ausbeute: 83%). Die Charakterisierung des Produkts erfolgte wie im Beispiel 1.

### Beispiel 6

100 g (0.09 Mol) einer perfluorierten Verbindung der Formel (II) mit n = 5 (erhalten durch Destillation eines bei +5°C hergestellten Acylfluorid-Gemisches und anschließende Verseifung und Decarboxylierung des dabei erhaltenen einzelnen Acylfluorids der Formel CF₃(CF₂)₂-O-[CF(CF₃)CF₂-O]ₙCF(CF₃)-COF mit n = 5) wurden mit 1.2 g AlCl₃ versetzt und auf eine Temperatur von 240°C erhitzt. Nach zweistündiger Reaktionszeit wurden aus dem Reaktionsgemisch über eine Kolonne bei 222°C 75 g einer leichtbeweglichen Flüssigkeit abdestilliert, die gemäß ¹⁹F-NMR und GC-Analyse die Formel (I) mit n = 4 hatte (Ausbeute: 86%). Die Charakterisierung des Produkts erfolgte wie im Beispiel 1.

### Beispiel 7

13500 g (3.65 Mol) eines Gemischs von Verbindungen mit der Formel (II) mit einem mittleren Molekulargewicht von 3700 gmol⁻¹ wurden auf 250°C erhitzt und 48 g AlCl₃, verteilt über einen Zeitraum von 16 Stunden, hinzugegeben. Man rührte für weitere 2 Stunden, filtrierte vom Feststoff und erhielt 12500 g an perfluorierten Verbindungen (I) (Ausbeute: 96%). Die Charakterisierung des Produkts erfolgte wie im Beispiel 1.

## Patentansprüche

1. Verfahren zur Herstellung von perfluorierten Ethern der Formel (I)
R-CF₂-CF₃ (I),
worin R den Rest CF₃(CF₂)₂-O-[CF(CF₃)CF₂-O]ₙ- bedeutet, in dem n eine ganze Zahl von 0-60 ist, aus Verbindungen der Formel (II)
R-[CF(CF₃)CF₂-O-]CHF-CF₃ (II),
dadurch gekennzeichnet, daß man die Verbindungen der Formel (II) in Gegenwart von mindestens einer der Verbindungen AlCl₃, AlBr₃, AlF₃ auf 150-350°C erhitzt.

2. Verfahren nach Anspruch 1, dadurch gekennzeichnet, daß man 1-100 Mol-% von mindestens einem der genannten Aluminiumhalogenide verwendet.

3. Verfahren nach Anspruch 1, dadurch gekennzeichnet, daß man 5-10 Mol-% von mindestens einem der genannten Aluminiumhalogenide verwendet.

4. Verfahren nach einem der Ansprüche 1-3, dadurch gekennzeichnet, daß man die Umsetzung bei 200-280°C durchführt.

5. Verfahren nach einem der Ansprüche 1-3, dadurch gekennzeichnet, daß man die Umsetzung bei 240-260°C durchführt.
